# EUROPÄISCHE PATENTANMELDUNG

(11) **EP 4 710 973 A1**
(43) Veröffentlichungstag der Anmeldung: **18.03.2026**
(21) Anmeldenummer: 25185414.7
(22) Anmeldetag: 26.06.2025
(51) Int. Cl.: A61M 16/00, A61M 16/16, A61M 11/00

(54) **VERFAHREN ZUM BETREIBEN EINES ATEMLUFTBEFEUCHTERS**

(30) Priorität: 12.09.2024 DE 102024126301
(71) Anmelder: Löwenstein Medical Technology S.A., 2557 Luxembourg (LU)
(72) Erfinder: Furthmueller, Jochen, 76287 Rheinstetten (DE); Schwaibold, Matthias, 76228 Karlsruhe (DE)
(74) Vertreter: Löwenstein Medical IP

(57) **Zusammenfassung**

Ein Atemluftbefeuchter (1) umfasst einen Atemlufteingang (3), einen Atemluftausgang (5) und ein elektrisches Heizelement (9). Der Atemluftbefeuchter (1) ist zwischen einem Befeuchtungsmodus und einem Vorbereitungsmodus zum Vorbereiten des Atemluftbefeuchters (1) auf einen Betrieb im Befeuchtungsmodus umschaltbar und ausgebildet, um im Befeuchtungsmodus Atemluft, die am Atemlufteingang (3) zugeführt wird, zu befeuchten, wobei die Atemluft mittels des Heizelements (9) temperiert wird, und im derart befeuchteten Zustand am Atemluftausgang (5) bereitzustellen. Ein Verfahren zum Betreiben des Atemluftbefeuchters (1) umfasst: wenn der Atemluftbefeuchter (1) ein- oder ausgeschaltet wird, Schalten des Atemluftbefeuchters (1) in den Vorbereitungsmodus und Betreiben des Atemluftbefeuchters (1) im Vorbereitungsmodus, um eine Abfuhr überschüssiger Wärme aufgrund eines dem Ein- oder Ausschalten vorangegangenen Betriebs des Atemluftbefeuchters (1) im Befeuchtungsmodus aus dem Inneren des Atemluftbefeuchters (1) zu ermöglichen, wobei das Heizelement (9) deaktiviert ist.

## Beschreibung

### Technisches Gebiet

Die Erfindung betrifft ein Verfahren zum Betreiben eines Atemluftbefeuchters. Des Weiteren betrifft die Erfindung eine Steuereinheit, ein Computerprogramm und ein computerlesbares Medium zum Ausführen des Verfahrens sowie einen Atemluftbefeuchter.

### Stand der Technik

Künstlich beatmete Patienten können manchmal unter zu trockenen Atemwegen leiden. In solchen Fällen kann die einzuatmende Atemluft mithilfe eines Atemluftbefeuchters zusätzlich befeuchtet und dabei mittels eines elektrischen Heizelements auf eine geeignete Temperatur gebracht werden. Somit kann eine Austrocknung der Schleimhäute vermieden oder zumindest abgemildert werden. Wird die Beatmungstherapie kurzzeitig unterbrochen, so kann es sein, dass die Atemluft im Inneren des Atemluftbefeuchters aufgrund des zwar ausgeschalteten, aber noch warmen Heizelements weiterhin passiv befeuchtet und/oder erwärmt wird. Dies kann dazu führen, dass die eingeatmete Atemluft bei den ersten Atemzügen nach dem Fortsetzen der Beatmungstherapie als unangenehm warm und/oder unangenehm feucht empfunden wird.

### Offenbarung der Erfindung

Eine Aufgabe der Erfindung kann darin gesehen werden, ein Verfahren zum Betreiben eines Atemluftbefeuchters zu schaffen, mit dem verhindert werden kann, dass die eingeatmete Atemluft beim Einschalten des Atemluftbefeuchters nach einer kurzzeitigen Betriebsunterbrechung als unangenehm warm und/oder unangenehm feucht empfunden wird. Eine weitere Aufgabe der Erfindung kann darin gesehen werden, eine Steuereinheit, ein Computerprogramm und ein computerlesbares Medium zum Ausführen eines solchen Verfahrens sowie einen entsprechenden Atemluftbefeuchter bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Verfahren zum Betreiben eines Atemluftbefeuchters, insbesondere für ein Beatmungsgerät. Der Atemluftbefeuchter umfasst einen Atemlufteingang, einen Atemluftausgang und ein elektrisches Heizelement. Der Atemluftbefeuchter ist zwischen einem Befeuchtungsmodus und einem Vorbereitungsmodus zum Vorbereiten des Atemluftbefeuchters auf einen Betrieb im Befeuchtungsmodus umschaltbar und ausgebildet, um im Befeuchtungsmodus Atemluft, die am Atemlufteingang zugeführt wird, zu befeuchten, wobei die Atemluft mittels des Heizelements temperiert wird (d. h. durch Steuern des Heizelements auf eine beim Einatmen als angenehm empfundene Temperatur gebracht wird), und im befeuchteten Zustand am Atemluftausgang bereitzustellen. Das Verfahren umfasst: wenn der Atemluftbefeuchter ein- oder ausgeschaltet wird, Schalten des Atemluftbefeuchters in den Vorbereitungsmodus und Betreiben des Atemluftbefeuchters im Vorbereitungsmodus, um eine Abfuhr überschüssiger Wärme aufgrund eines dem Ein- oder Ausschalten vorangegangenen Betriebs des Atemluftbefeuchters im Befeuchtungsmodus aus dem Inneren des Atemluftbefeuchters zu ermöglichen (oder zu beschleunigen). Beim Betreiben des Atemluftbefeuchters im Vorbereitungsmodus ist das Heizelement deaktiviert.

Anders ausgedrückt kann der Atemluftbefeuchter jedes Mal, wenn der Atemluftbefeuchter ein- oder ausgeschaltet wird, genauer gesagt jedes Mal, wenn erkannt wird, dass der Atemluftbefeuchter ein- oder ausgeschaltet wird (oder werden soll), automatisch für eine bestimmte Zeit im Vorbereitungsmodus betrieben werden, um eine ausreichende passive und/oder aktive Abkühlung des Heizelements und/oder der Luft im Inneren des Atemluftbefeuchters vor dem erneuten Aktivieren des Befeuchtungsmodus, beispielsweise vor dem Fortsetzen einer kurzzeitig (z. B. für weniger als 10 Minuten, weniger als 5 Minuten oder weniger als 3 Minuten) unterbrochenen Beatmungstherapie, zu ermöglichen. Alternativ kann das Schalten in den Vorbereitungsmodus temperaturabhängig erfolgen. In diesem Fall ist es möglich, dass der Atemluftbefeuchter beim Ein- oder Ausschalten nur dann in den Vorbereitungsmodus geschaltet wird, wenn eine bestimmte Temperatur im Inneren des Atemluftbefeuchters erreicht wurde (siehe weiter unten).

Das Abführen der überschüssigen Wärme kann passiv und/oder aktiv, beispielsweise durch entsprechendes Steuern eines oder mehrerer elektropneumatischer Aktoren, erfolgen.

Es ist möglich, dass der Atemluftbefeuchter für eine fest vorgegebene Betriebsdauer (z. B. zwischen 10 Sekunden und 5 Minuten) im Vorbereitungsmodus betrieben wird. Alternativ kann die Betriebsdauer abhängig von einer aktuellen (gemessenen und/oder geschätzten) Temperatur im Inneren des Atemluftbefeuchters vorgegeben werden.

Der Betrieb des Atemluftbefeuchters im Vorbereitungsmodus kann sich auch nur darauf beschränken, dass ein Schalten des Atemluftbefeuchters in den Befeuchtungsmodus verhindert wird und/oder eine Aktivierung des Heizelements verhindert wird.

Bei dem Befeuchtungsmodus kann es sich - im Gegensatz zum Vorbereitungsmodus - um eine Hauptbetriebsart des Atemluftbefeuchters handeln. Der Atemluftbefeuchter kann im Vorbereitungsmodus anders als im Befeuchtungsmodus betrieben werden. Prinzipiell kann der Atemluftbefeuchter im Vorbereitungsmodus so betrieben werden, dass eine Wahrscheinlichkeit, mit der die am Atemluftausgang bereitgestellte Atemluft kurz nach dem Einschalten des Atemluftbefeuchters beim Einatmen als unangenehm warm und/oder unangenehm feucht empfunden wird, im Vergleich zu einem entsprechenden Betrieb des Atemluftbefeuchters im Befeuchtungsmodus in signifikanter Weise verringert wird.

Es ist möglich, dass das Heizelement bei jedem Einschalten des Atemluftbefeuchters und/oder bei jedem Schalten des Atemluftbefeuchters in den Befeuchtungsmodus aktiviert wird und/oder bei jedem Ausschalten des Atemluftbefeuchters und/oder bei jedem Schalten des Atemluftbefeuchters in den Vorbereitungsmodus deaktiviert wird. Anders ausgedrückt kann das Heizelement während des (gesamten) Betriebs des Atemluftbefeuchters im Befeuchtungsmodus aktiviert sein und/oder während des (gesamten) Betriebs des Atemluftbefeuchters im Vorbereitungsmodus deaktiviert sein. Das Heizelement kann im aktivierten Zustand kontinuierlich eingeschaltet sein. Dabei kann eine Heizleistung des Heizelements in geeigneter Weise variiert werden, um die Atemluft zu temperieren, beispielsweise durch Variieren eines durch das Heizelement fließenden elektrischen Stroms und/oder einer am Heizelement anliegenden elektrischen Spannung (z. B. durch Pulsweitenmodulation). Zusätzlich oder alternativ kann die (durchschnittliche) Heizleistung des Heizelements im aktivierten Zustand durch mehrmaliges abwechselndes Ein- und Ausschalten des Heizelements gesteuert werden. Hingegen kann das Heizelement im deaktivierten Zustand kontinuierlich ausgeschaltet, d. h. von einer Stromversorgung elektrisch entkoppelt sein, sodass die Atemluft nicht - oder zumindest nicht gezielt oder aktiv - mittels des Heizelements erwärmt wird.

Wird der Atemluftbefeuchter im Befeuchtungsmodus betrieben, so ist es möglich, dass der Atemluftbefeuchter erst dann wieder in den Vorbereitungsmodus geschaltet werden kann, wenn der Atemluftbefeuchter vorher ausgeschaltet wird. Anders ausgedrückt kann der Atemluftbefeuchter, nachdem er einmal im Vorbereitungsmodus betrieben wurde, erst nach einem erneuten Ein- bzw. Ausschalten des Atemluftbefeuchters erneut in den Vorbereitungsmodus schaltbar sein. Beispielsweise kann der Betrieb des Atemluftbefeuchters im Befeuchtungsmodus nur durch Ausschalten des Atemluftbefeuchters - und nicht durch Schalten des Atemluftbefeuchters in den Vorbereitungsmodus - unterbrochen werden.

Der Atemluftbefeuchter kann beim Ausschalten stromlos geschaltet werden. Anders ausgedrückt kann der Atemluftbefeuchter beim Ausschalten von einer Stromversorgung, beispielsweise über ein Stromnetz und/oder eine Batterie, elektrisch entkoppelt werden. Alternativ kann der Atemluftbefeuchter beim Ausschalten in einen Stromsparbetrieb geschaltet werden. Der (ausgeschaltete) Atemluftbefeuchter kann im Stromsparbetrieb weiterhin mit Strom versorgt werden (beispielsweise über eine zusätzliche Batterie), dabei jedoch deutlich weniger Energie als im eingeschalteten Zustand verbrauchen. Insbesondere kann im Stromsparbetrieb das Heizelement deaktiviert sein. Dementsprechend ist es möglich, dass der Stromsparbetrieb beim Einschalten des Atemluftbefeuchters wieder beendet wird, beispielsweise wenn der Atemluftbefeuchter beim Einschalten in den Befeuchtungsmodus (statt in den Vorbereitungsmodus) geschaltet wird. Alternativ kann der Stromsparbetrieb beim Einschalten aufrechterhalten werden, wenn der Atemluftbefeuchter beim Einschalten in den Vorbereitungsmodus geschaltet wird. Anders ausgedrückt kann der (eingeschaltete) Atemluftbefeuchter im Vorbereitungsmodus deutlich weniger Energie als im Befeuchtungsmodus verbrauchen.

Unter "Atemluft" kann ein einzelnes Atemgas oder ein Gemisch aus mehreren Atemgasen zum Beatmen eines Patienten verstanden werden. Unter "Atemgas" kann beispielsweise Kohlendioxid, Sauerstoff, Stickstoff, Wasserdampf oder Narkosegas verstanden werden.

Unter einer Befeuchtung der Atemluft kann eine gezielte Vermischung der Atemluft mit Wasserdampf und/oder Wassertröpfchen in Kombination mit einer gezielten Erwärmung der Atemluft verstanden werden.

Das Verfahren kann beispielsweise computerimplementiert sein.

Ein zweiter Aspekt der Erfindung betrifft eine Steuereinheit, die konfiguriert ist, um das vor- und nachstehend beschriebene Verfahren auszuführen.

Die Steuereinheit kann Mittel zur Datenverarbeitung umfassen. Die Mittel zur Datenverarbeitung können als Hard- und/oder Software implementiert sein und/oder einen Prozessor umfassen. Der Prozessor kann konfiguriert sein, um das (computerimplementierte) Verfahren auszuführen. Zusätzlich zum Prozessor kann die Steuereinheit mindestens eines der folgenden Mittel zur Datenverarbeitung umfassen: einen Speicher, ein Bussystem zur Datenkommunikation zwischen dem Speicher und dem Prozessor, eine Datenkommunikationsschnittstelle zur drahtlosen und/oder drahtgebundenen Datenkommunikation mit Peripheriegeräten. Alternativ kann die Steuereinheit ausschließlich als Hardware, beispielsweise in Form eines ASIC- oder FPGA-Bausteins, implementiert sein.

Es wird darauf hingewiesen, dass Merkmale des vor- und nachstehend beschriebenen Verfahrens auch Merkmale der Steuereinheit sein können (und umgekehrt).

Ein dritter Aspekt der Erfindung betrifft einen Atemluftbefeuchter, insbesondere für ein Beatmungsgerät. Der Atemluftbefeuchter umfasst einen Atemlufteingang, einen Atemluftausgang, ein elektrisches Heizelement und eine Steuereinheit, wie sie vor- und nachstehend beschrieben wird. Der Atemluftbefeuchter ist zwischen einem Befeuchtungsmodus und einem Vorbereitungsmodus zum Vorbereiten des Atemluftbefeuchters auf einen Betrieb im Befeuchtungsmodus umschaltbar und ausgebildet, um im Befeuchtungsmodus Atemluft, die am Atemlufteingang zugeführt wird, zu befeuchten, wobei die Atemluft mittels des Heizelements temperiert wird, und im befeuchteten Zustand am Atemluftausgang bereitzustellen.

Der Atemlufteingang kann beispielsweise an ein Beatmungsgerät zum invasiven und/oder nicht invasiven Beatmen eines Patienten anschließbar sein, um eine Befeuchtung der vom Beatmungsgerät bereitgestellten Atemluft zu ermöglichen, bevor die Atemluft vom Patienten eingeatmet wird. Unter "Beatmungsgerät" kann auch ein Anästhesiegerät oder ein Hustenunterstützungsgerät zum Unterstützen eines Patienten beim Husten, auch Insufflator-Exsufflator genannt, verstanden werden.

Der Atemluftausgang kann beispielsweise über einen oder mehrere Beatmungsschläuche und/oder über eine Patientenschnittstelle (beispielsweise eine Maske, eine Nasenkanüle und/oder einen Tubus) an den Atemapparat des Patienten anschließbar sein, sodass eine Beatmung des Patienten mit der befeuchteten Atemluft möglich ist.

Der Atemluftbefeuchter kann ausgebildet sein, um die Atemluft zu befeuchten, indem die Atemluft zum einen mit Wasserdampf und/oder Wassertröpfchen vermischt wird und zum anderen durch direkten und/oder indirekten Kontakt mit dem Heizelement temperiert wird. Beispielsweise kann der Atemluftbefeuchter ausgebildet sein, um Wasserdampf zum Befeuchten der Atemluft durch Erwärmen von Wasser mittels des Heizelements und/oder Wassertröpfchen zum Befeuchten der Atemluft, insbesondere ein Aerosol, durch Zerstäuben von Wasser mittels eines Zerstäubers, beispielsweise eines piezoelektrischen Ultraschallzerstäubers, zu erzeugen.

Die Steuereinheit kann beispielsweise konfiguriert sein, um das Heizelement und/oder einen oder mehrere elektropneumatische Aktoren beim Betrieb des Atemluftbefeuchters im Befeuchtungsmodus so zu steuern, dass sich eine tatsächliche Temperatur der am Atemluftausgang bereitgestellten Atemluft einer gewünschten Komforttemperatur annähert, bei der die Atemluft beim Einatmen als angenehm warm empfunden wird. Die Komforttemperatur kann beispielsweise zwischen 30 °C und 38 °C liegen.

In entsprechender Weise kann die Steuereinheit konfiguriert sein, um das Heizelement und/oder einen oder mehrere elektropneumatische Aktoren beim Betrieb des Atemluftbefeuchters im Befeuchtungsmodus so zu steuern, dass sich eine tatsächliche relative Luftfeuchtigkeit der am Atemluftausgang bereitgestellten Atemluft bezogen auf eine jeweilige Umgebungstemperatur einer gewünschten relativen Luftfeuchtigkeit annähert, bei der die Atemluft beim Einatmen als angenehm feucht empfunden wird.

Unter dem Begriff "elektrisches Heizelement" kann allgemein ein elektrisch steuerbares Heizelement, insbesondere ein Heizwiderstand, verstanden werden. Das Heizelement kann beispielsweise einen Heizstab, eine Heizwendel und/oder eine Heizplatte umfassen. Das Heizelement kann ausgebildet sein, um die Atemluft direkt und/oder indirekt, beispielsweise durch Erwärmen einer Flüssigkeit, mit der die Atemluft beim Strömen durch den Atemluftbefeuchter in Kontakt kommt, zu temperieren.

Der Atemluftbefeuchter kann als ein eigenständiges Gerät und/oder als eine Komponente eines medizintechnischen Geräts, insbesondere eines Beatmungsgeräts, ausgeführt sein.

Weitere Aspekte der Erfindung betreffen ein Computerprogramm und ein computerlesbares Medium, auf dem das Computerprogramm gespeichert ist.

Das Computerprogramm umfasst Befehle, die die vor- und nachstehend beschriebene Steuereinheit, beispielsweise einen Prozessor der Steuereinheit, beim Ausführen des Computerprogramms durch die Steuereinheit veranlassen, das vor- und nachstehend beschriebene Verfahren auszuführen.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*), ein RAM (*random-access memory*), ein ROM (*read-only memory*), ein EPROM (*erasable programmable read-only memory*), ein EEPROM (*electrically erasable programmable read-only memory*), ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale des vor- und nachstehend beschriebenen Verfahrens auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann das Verfahren ferner umfassen, wenn der Atemluftbefeuchter eingeschaltet wird: Schalten des Atemluftbefeuchters in den Befeuchtungsmodus im Anschluss an den Betrieb des Atemluftbefeuchters im Vorbereitungsmodus und Betreiben des Atemluftbefeuchters im Befeuchtungsmodus, beispielsweise so lange, bis der Atemluftbefeuchter wieder ausgeschaltet wird. Anders ausgedrückt kann der Atemluftbefeuchter im Anschluss an den Vorbereitungsmodus automatisch in den Befeuchtungsmodus geschaltet werden. Der Atemluftbefeuchter kann beispielsweise so lange im Vorbereitungsmodus betrieben werden, bis (beispielsweise von einer Steuereinheit des Atemluftbefeuchters) erkannt wird, dass ein bestimmtes Umschaltkriterium zum Umschalten des Atemluftbefeuchters in den Befeuchtungsmodus erfüllt wird. Ein solches Umschaltkriterium kann beispielsweise der Ablauf einer vorgegebenen Betriebsdauer des Atemluftbefeuchters im Vorbereitungsmodus ab einem Zeitpunkt, zu dem der Atemluftbefeuchters ein- oder ausgeschaltet wird, und/oder das Erreichen einer vorgegebenen Temperatur im Inneren des Atemluftbefeuchters sein. Es ist möglich, dass der Atemluftbefeuchter bei jedem Einschalten nur ein einziges Mal in den Vorbereitungsmodus geschaltet wird und, nachdem er in den Befeuchtungsmodus geschaltet wurde, erst dann wieder in den Vorbereitungsmodus schaltbar ist, wenn er ausgeschaltet oder aus- und wieder eingeschaltet wird.

Gemäß einer Ausführungsform kann das Verfahren ferner umfassen, wenn der Atemluftbefeuchter ein- oder ausgeschaltet wird: Empfangen eines Temperaturwerts, der eine aktuelle Temperatur der Atemluft im Inneren des Atemluftbefeuchters anzeigt; Vergleichen des Temperaturwerts mit einem Schwellenwert, der eine Temperaturschwelle anzeigt, bei der die Atemluft beim Einatmen gerade noch als angenehm empfunden wird; wenn der Temperaturwert den Schwellenwert überschreitet, Schalten des Atemluftbefeuchters in den Vorbereitungsmodus und Betreiben des Atemluftbefeuchters im Vorbereitungsmodus. Anders ausgedrückt kann das Schalten des Atemluftbefeuchters in den Vorbereitungsmodus temperaturgesteuert erfolgen. Dies hat den Vorteil, dass der Atemluftbefeuchter nur dann im Vorbereitungsmodus betrieben wird, wenn dies tatsächlich erforderlich ist. Der Temperaturwert kann ein Messwert, d. h. ein mittels eines Temperatursensors bestimmter Wert, und/oder ein Schätzwert sein.

Zusätzlich oder alternativ kann - wenn der Temperaturwert den Schwellenwert nicht überschreitet - verhindert werden, dass der Atemluftbefeuchter in den Vorbereitungsmodus geschaltet wird.

Gemäß einer Ausführungsform kann das Verfahren ferner umfassen, wenn der Atemluftbefeuchter eingeschaltet wird: wenn der Temperaturwert den Schwellenwert nicht überschreitet, Schalten des Atemluftbefeuchters in den Befeuchtungsmodus und Betreiben des Atemluftbefeuchters im Befeuchtungsmodus, beispielsweise so lange, bis der Atemluftbefeuchter wieder ausgeschaltet wird. Anders ausgedrückt kann der Atemluftbefeuchter beim Einschalten direkt in den Befeuchtungsmodus geschaltet werden, sofern die Atemluft im Inneren des Atemluftbefeuchters seit dem letzten Betrieb im Befeuchtungsmodus ausreichend abgekühlt ist. Somit kann eine unnötige Aktivierung des Vorbereitungsmodus vermieden werden.

Gemäß einer Ausführungsform kann der Temperaturwert in mehreren aufeinanderfolgenden Zeitschritten empfangen und in jedem Zeitschritt mit dem Schwellenwert verglichen werden. Dabei kann der Atemluftbefeuchter so lange im Vorbereitungsmodus betrieben werden, bis der Temperaturwert den Schwellenwert in einem der Zeitschritte nicht mehr überschreitet. Dies ermöglicht ein automatisches, temperaturabhängiges Beenden des Vorbereitungsmodus, beispielsweise kombiniert mit einem automatischen Umschalten des Atemluftbefeuchters vom Vorbereitungsmodus in den Befeuchtungsmodus. Umgekehrt kann beispielsweise ein automatisches Umschalten des Atemluftbefeuchters vom Befeuchtungsmodus in den Vorbereitungsmodus - wie weiter oben angemerkt - als Reaktion auf ein Ausschalten oder ein Aus- und wieder Einschalten des Atemluftbefeuchters erfolgen.

Gemäß einer Ausführungsform kann das Verfahren ferner umfassen: Berechnen des Temperaturwerts unter Verwendung eines mathematischen Modells des Atemluftbefeuchters. Dabei kann mindestens ein Eingabewert empfangen und in das mathematische Modell eingegeben werden. Das mathematische Modell kann konfiguriert sein, um den mindestens einen Eingabewert in den Temperaturwert umzuwandeln und den Temperaturwert als einen Ausgabewert auszugeben. Der mindestens eine Eingabewert kann beispielsweise mindestens eine der folgenden Größen anzeigen:
- eine (aktuelle) Heizleistung des Heizelements;
- einen (aktuellen) Füllstand eines Behälters zum Speichern einer Flüssigkeit zum Befeuchten der Atemluft, beispielsweise von Wasser oder einer wasserhaltigen Flüssigkeit;
- eine (beispielsweise durchschnittliche) Dauer mindestens einer dem Ein- oder Ausschalten vorangegangenen Aufheizphase, in der das Heizelement aktiviert war;
- eine (beispielsweise durchschnittliche) Dauer mindestens einer dem Ein- oder Ausschalten vorangegangenen Abkühlphase, in der das Heizelement deaktiviert war;
- eine (aktuelle) Umgebungstemperatur in der Umgebung des Atemluftbefeuchters;
- eine (aktuelle) relative Luftfeuchtigkeit bezogen auf eine (aktuelle) Umgebungstemperatur in der Umgebung des Atemluftbefeuchters;
- einen (aktuellen) Umgebungsdruck in der Umgebung des Atemluftbefeuchters.

Unter "Eingabewert" kann ein die jeweilige(n) Größe(n) quantifizierender gemessener und/oder geschätzter Wert verstanden werden. Der mindestens eine Eingabewert kann beispielsweise in mehreren aufeinanderfolgenden Zeitschritten empfangen und in das mathematische Modell eingegeben werden. Dabei kann das mathematische Modell in jedem Zeitschritt den Temperaturwert als einen dem Eingabewert oder den Eingabewerten des jeweiligen Zeitschritts zugeordneten Ausgabewert ausgeben. Diese Ausführungsform ermöglicht es, den Temperaturwert auch ohne Zuhilfenahme eines gesonderten Temperatursensors mit ausreichender Genauigkeit zu bestimmen.

Gemäß einer Ausführungsform kann das Betreiben des Atemluftbefeuchters im Vorbereitungsmodus umfassen: Erzeugen eines Steuersignals zum Steuern mindestens eines elektropneumatischen Aktors, um einen Luftstrom zum Abführen der überschüssigen Wärme durch den Atemlufteingang und/oder durch den Atemluftausgang und/oder durch mindestens eine vom Atemlufteingang und vom Atemluftausgang getrennte Belüftungsöffnung des Atemluftbefeuchters zu erzeugen.

Die Belüftungsöffnung kann beispielsweise in einem Gehäuse des Atemluftbefeuchters und/oder in einem Gehäuse eines Beatmungsgeräts ausgebildet sein. Unter dem Begriff "elektropneumatischer Aktor" kann vor- und nachstehend allgemein ein elektrisch steuerbarer Aktor zum Steuern eines Luftstroms verstanden werden. Beispielsweise kann der elektropneumatische Aktor ein Gebläse und/oder ein elektrisch steuerbares Ventil, beispielsweise ein Magnetventil, sein. Mithilfe einer derartigen aktiven Wärmeabfuhr kann die Abkühlung des Atemluftbefeuchters im Vergleich zu einer Ausführungsform mit rein passiver Wärmeabfuhr deutlich beschleunigt werden.

Gemäß einer Ausführungsform kann das Betreiben des Atemluftbefeuchters im Vorbereitungsmodus umfassen: Erzeugen eines Steuersignals zum Steuern mindestens eines elektropneumatischen Aktors, um zu verhindern, dass die Atemluft am Atemluftausgang bereitgestellt wird. Auf diese Weise kann verhindert werden, dass möglicherweise zu warme und/oder zu feuchte Atemluft vom Patienten eingeatmet wird.

Gemäß einer Ausführungsform kann das Betreiben des Atemluftbefeuchters im Vorbereitungsmodus umfassen: Erzeugen eines Steuersignals zum Steuern mindestens eines elektropneumatischen Aktors, um die Atemluft am Atemluftausgang mit einem (deutlich) geringeren Druck und/oder einem (deutlich) geringeren Volumenstrom und/oder einer anderen Strömungsrichtung als beim Betreiben des Atemluftbefeuchters im Befeuchtungsmodus bereitzustellen. Aufgrund des geringeren Drucks und/oder des geringeren Volumenstroms kann verhindert werden, dass die Atemluft, auch wenn sie noch nicht ausreichend abgekühlt sein sollte, beim Einatmen als unangenehm warm und/oder unangenehm feucht empfunden wird. Der Volumenstrom bzw. der Druck im Vorbereitungsmodus kann beispielsweise um mindestens 5 %, mindestens 10 %, mindestens 20 % oder mindestens 50 % vom Volumenstrom bzw. Druck im Befeuchtungsmodus abweichen. Die Strömungsrichtung im Vorbereitungsmodus kann der Strömungsrichtung im Befeuchtungsmodus entgegengesetzt sein. Anders ausgedrückt ist es möglich, dass die Atemluft im Vorbereitungsmodus am Atemluftausgang eingesaugt statt - wie im Befeuchtungsmodus - ausgeblasen wird. Auf diese Weise kann verhindert werden, dass die möglicherweise zu warme und/oder zu feuchte Atemluft vom Patienten eingeatmet wird.

Gemäß einer Ausführungsform kann das Betreiben des Atemluftbefeuchters im Vorbereitungsmodus umfassen: Erzeugen eines Steuersignals zum Steuern mindestens eines elektropneumatischen Aktors, sodass ein Volumenstrom der am Atemluftausgang bereitgestellten Atemluft einem teilweise oder kontinuierlich ansteigenden Volumenstromverlauf folgt und/oder ein Druck der am Atemluftausgang bereitgestellten Atemluft einem teilweise oder kontinuierlich ansteigenden Druckverlauf folgt. Anders ausgedrückt kann der Volumenstrom oder der Druck oder können der Volumenstrom und der Druck im Vorbereitungsmodus verzögert, beispielsweise stufenweise, erhöht werden. Ein derartiger (zumindest teilweise) ansteigender Verlauf ermöglicht es dem Patienten, eine an den Atemluftausgang angeschlossene Maske oder Nasenkanüle rechtzeitig abzunehmen, falls die Atemluft aus der Maske bzw. Nasenkanüle beim Einatmen als unangenehm warm und/oder unangenehm feucht empfunden werden sollte. Hingegen kann der elektromagnetische Aktor oder können die elektromagnetischen Aktoren im Befeuchtungsmodus beispielsweise so gesteuert werden, dass ein konstanter Verlauf des Volumenstroms und/oder des Drucks resultiert.

Beispielsweise kann beim Fortsetzen einer Highflowtherapie zunächst ein reduzierter Atemluftstrom am Atemluftausgang bereitgestellt werden. Dazu kann ein entsprechendes Gebläse besonders langsam wieder angefahren werden, sodass der resultierende Atemluftstrom für einen geeigneten Zeitraum geringer bleibt als eigentlich für die Highflowtherapie vorgesehen. Die Vermischung der Atemluft mit der Umgebungsluft an der jeweiligen Patientenschnittstelle bewirkt dabei, dass das eingeatmete Luftgemisch eine kritische Temperatur nicht überschreitet.

Gemäß einer Ausführungsform kann das Betreiben des Atemluftbefeuchters im Vorbereitungsmodus umfassen: Erzeugen eines Steuersignals zum Steuern mindestens eines elektropneumatischen Aktors, sodass ein Volumenstrom der am Atemluftausgang bereitgestellten Atemluft am Ende des Betriebs des Atemluftbefeuchters im Vorbereitungsmodus einen Endwert erreicht, der gleich einem oder kleiner als ein Volumenstromsollwert ist, den der Volumenstrom zu Beginn eines anschließenden Betriebs des Atemluftbefeuchters im Befeuchtungsmodus haben soll. Beispielsweise kann der Volumenstrom im Vorbereitungsmodus allmählich bis auf den Volumenstromsollwert oder bis auf einen geringfügig (beispielsweise höchstens 10 % oder höchstens 5 %) kleineren Wert als der Volumenstromsollwert erhöht werden. Somit können möglicherweise als unkomfortabel empfundene Schwankungen des Volumenstroms beim Umschalten vom Vorbereitungs- in den Befeuchtungsmodus vermieden werden.

Gemäß einer Ausführungsform kann das Betreiben des Atemluftbefeuchters im Vorbereitungsmodus umfassen: Erzeugen eines Steuersignals zum Steuern mindestens eines elektropneumatischen Aktors, sodass ein Druck der am Atemluftausgang bereitgestellten Atemluft am Ende des Betriebs des Atemluftbefeuchters im Vorbereitungsmodus einen Endwert erreicht, der gleich einem oder kleiner als ein Drucksollwert ist, den der Druck zu Beginn eines anschließenden Betriebs des Atemluftbefeuchters im Befeuchtungsmodus haben soll. Beispielsweise kann der Druck im Vorbereitungsmodus allmählich bis auf den Drucksollwert oder bis auf einen geringfügig (beispielsweise höchstens 10 % oder höchstens 5 %) kleineren Wert als der Drucksollwert erhöht werden. Somit können möglicherweise als unkomfortabel empfundene Schwankungen des Drucks beim Umschalten vom Vorbereitungs- in den Befeuchtungsmodus vermieden werden.

Gemäß einer Ausführungsform kann der Atemluftbefeuchter ferner einen mittels einer elektrischen Schlauchheizung beheizbaren Beatmungsschlauch zum Anschließen an den Atemluftausgang umfassen. Der Beatmungsschlauch kann im betriebsfähigen Zustand des Atemluftbefeuchters an den Atemluftausgang angeschlossen sein. Der Atemluftbefeuchter kann ferner ausgebildet sein, um im Befeuchtungsmodus die durch den Beatmungsschlauch strömende Atemluft mittels der Schlauchheizung zusätzlich zu temperieren. Somit kann beispielsweise verhindert werden, dass die Atemluft auf ihrem Weg im Beatmungsschlauch vom Atemluftausgang zum Atemapparat eines beatmeten Patienten zu stark abkühlt. In diesem Fall kann das Verfahren ferner umfassen: Verhindern einer Aktivierung der Schlauchheizung, solange der Atemluftbefeuchter im Vorbereitungsmodus betrieben wird. Auf diese Weise kann eine unerwünschte zusätzliche Erwärmung der im Vorbereitungsmodus durch den Beatmungsschlauch strömenden und möglicherweise bereits unangenehm feuchten und/oder unangenehm warmen Atemluft vermieden werden.

Gemäß einer Ausführungsform kann ferner ein Warnhinweis zum Warnen eines Benutzers des Atemluftbefeuchters erzeugt werden, wenn der Atemluftbefeuchter in den Vorbereitungsmodus geschaltet wird. Der Warnhinweis kann beispielsweise ein optischer und/oder akustischer und/oder haptischer Hinweis sein. Der Warnhinweis kann über den Atemluftbefeuchter selbst und/oder über ein Peripheriegerät, das mit dem Atemluftbefeuchter drahtlos und/oder drahtgebunden zur Datenkommunikation verbunden ist (z. B. über das Internet), wiedergegeben werden. Bei einem solchen Peripheriegerät kann es sich beispielsweise um ein medizintechnisches Gerät (insbesondere ein Beatmungsgerät), ein Smartphone, eine Smartwatch, ein Tablet, einen Laptop oder einen PC handeln. Beispielsweise kann der Benutzer mithilfe des Warnhinweises dazu aufgefordert werden, eine an den Atemluftausgang angeschlossene Maske oder Nasenkanüle abzunehmen, sodass ein Kontakt der Atemwege des Benutzers mit der möglicherweise zu warmen und/oder zu feuchten Atemluft vermieden wird.

Gemäß einer Ausführungsform kann eine Aktivierung des Heizelements verhindert werden, solange der Atemluftbefeuchter im Vorbereitungsmodus betrieben wird. Dazu kann das Heizelement beispielsweise mittels eines gesonderten Schalters von einer Stromversorgung getrennt werden. Somit kann beispielsweise eine versehentliche Aktivierung des Heizelements während des Betriebs des Atemluftbefeuchters im Vorbereitungsmodus vermieden werden.

Gemäß einer Ausführungsform kann der Atemluftbefeuchter ferner umfassen: einen Atemluftkanal zum Leiten der Atemluft zwischen dem Atemlufteingang und dem Atemluftausgang; einen Behälter zum Speichern einer Flüssigkeit zum Befeuchten der Atemluft, beispielsweise von Wasser oder einer wasserhaltigen Flüssigkeit. Dabei kann das Heizelement ausgebildet sein, um die Flüssigkeit aus dem Behälter zu erwärmen. Dementsprechend kann der Atemluftbefeuchter so ausgebildet sein, dass die Atemluft beim Strömen durch den Atemluftkanal in Kontakt mit der Flüssigkeit im verdampften und/oder erwärmten Zustand kommt. Dadurch kann die Atemluft befeuchtet werden, bevor sie am Atemluftausgang austritt.

Zusätzlich kann der Atemluftbefeuchter einen oder mehrere elektropneumatische Aktoren zum Steuern eines Atemluftstroms durch den Atemluftkanal umfassen.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt einen Atemluftbefeuchter gemäß einer Ausführungsform der Erfindung.
Fig. 2 zeigt ein Ablaufdiagramm eines Verfahrens gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

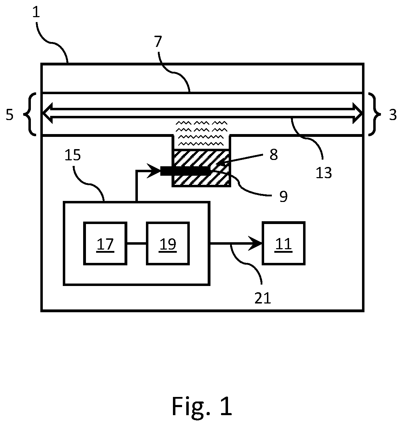

Fig. 1 zeigt einen Atemluftbefeuchter 1 zur Verwendung in Kombination mit einem Beatmungsgerät. In diesem Beispiel umfasst der Atemluftbefeuchter 1 einen Atemlufteingang 3 zum Anschließen des Beatmungsgeräts, sodass Atemluft aus dem Beatmungsgerät ins Innere des Atemluftbefeuchters 1 strömen kann, einen Atemluftausgang 5, einen Atemluftkanal 7 zum Leiten der Atemluft zwischen dem Atemlufteingang 3 und dem Atemluftausgang 5, einen Behälter 8 zum Speichern einer Flüssigkeit zum Befeuchten der Atemluft (beispielsweise von Wasser oder einer wasserhaltigen Flüssigkeit) und ein elektrisches Heizelement 9 zum Erwärmen der Flüssigkeit aus dem Behälter 8. Der Atemluftbefeuchter 1 ist so ausgebildet, dass die Atemluft beim Strömen durch den Atemluftkanal 7 vom Atemlufteingang 3 zum Atemluftausgang 5 in Kontakt mit der Flüssigkeit im verdampften und erwärmten Zustand kommt, wodurch die Atemluft befeuchtet (und dabei temperiert) wird, bevor sie am Atemluftausgang 5 austritt. Beispielsweise kann die Atemluft beim Befeuchten durch entsprechendes Steuern des Heizelements 9 auf eine bestimmte Komforttemperatur gebracht werden, bei der die Atemluft beim Einatmen als angenehm warm empfunden wird. Der Atemluftausgang 5 kann über einen oder mehrere Beatmungsschläuche und/oder eine geeignete Patientenschnittstelle wie beispielweise eine Beatmungsmaske, eine Nasenkanüle oder einen Tubus an den Atemapparat eines Patienten angeschlossen sein, sodass eine Beatmung des Patienten mit der Atemluft aus dem Atemluftbefeuchter 1 möglich ist.

Zusätzlich kann der Atemluftbefeuchter 1 mindestens einen elektropneumatischen Aktor 11 zum Steuern eines Atemluftstroms 13 durch den Atemluftkanal 7 umfassen. Der Aktor 11 kann beispielsweise ein Gebläse und/oder ein elektrisch steuerbares Ventil sein. Es ist möglich, dass der Aktor 11 (oder mindestens einer der Aktoren 11) in einem Gehäuse des Atemluftbefeuchters angeordnet ist. Der Aktor 11 (oder mindestens einer der Aktoren 11) kann jedoch auch in einem Gehäuse des Beatmungsgeräts angeordnet sein.

Ferner umfasst der Atemluftbefeuchter 1 eine Steuereinheit 15 zum Steuern eines Betriebs des Atemluftbefeuchters 1. Die Steuereinheit 15 kann als eine Komponente des Atemluftbefeuchters 1 ausgeführt sein und/oder im Gehäuse des Atemluftbefeuchters 1 angeordnet sein. Die Steuereinheit 15 kann jedoch auch als eine Komponente des Beatmungsgeräts ausgeführt sein und/oder im Gehäuse des Beatmungsgeräts angeordnet sein.

Die Steuereinheit 15 kann konfiguriert sein, um den Atemluftbefeuchter 1 zwischen einem Befeuchtungsmodus und einem Vorbereitungsmodus zum Vorbereiten des Atemluftbefeuchters 1 auf einen Betrieb im Befeuchtungsmodus umzuschalten. Der Atemluftbefeuchter 1 kann ausgebildet sein, um im Befeuchtungsmodus Atemluft, die am Atemlufteingang 3 - hier über das Beatmungsgerät - zugeführt wird, zu befeuchten, wobei die Atemluft mittels des Heizelements 9 temperiert wird, und im befeuchteten Zustand am Atemluftausgang 5 als einen Atemluftstrom 13 bereitzustellen, beispielsweise durch entsprechendes Steuern des Aktors 11 (oder der Aktoren 11).

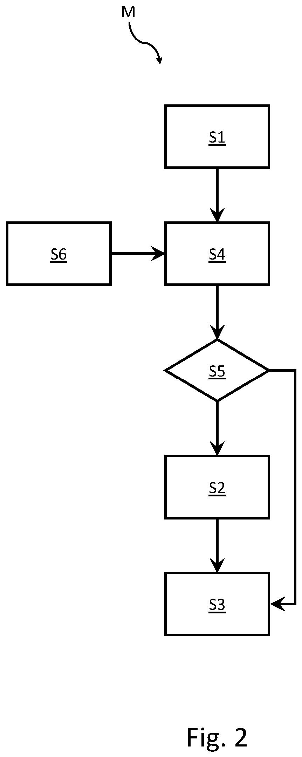

Die Steuereinheit 15 kann beispielsweise einen Prozessor 17 und einen Speicher 19 umfassen, in dem ein Computerprogramm zum Betreiben des Atemluftbefeuchters 1 gespeichert sein kann. Der Prozessor 17 kann konfiguriert sein, um durch Ausführen des Computerprogramms folgendes Verfahren M (siehe Fig. 2) auszuführen.

In einem ersten Schritt S1 wird ein Ein- oder Ausschalten des Atemluftbefeuchters 1 erkannt, beispielsweise ein manuelles Ein- oder Ausschalten, wenn der Patient die Beatmungstherapie aus irgendeinem Grund für eine kurze Dauer (beispielsweise für eine Dauer zwischen 30 Sekunden und 3 Minuten) unterbrochen hat oder unterbrechen will.

Als Reaktion auf das Erkennen des Ein- oder Ausschaltens im Schritt S1 wird der Atemluftbefeuchter 1 in einem Schritt S2 in den Vorbereitungsmodus geschaltet und für eine bestimmte Betriebsdauer im Vorbereitungsmodus betrieben, um überschüssige Wärme aufgrund eines dem Ein- oder Ausschalten vorangegangenen Betriebs des Atemluftbefeuchters 1 im Befeuchtungsmodus aus dem Inneren des Atemluftbefeuchters 1, beispielsweise aus dem Atemluftkanal 7, aktiv und/oder passiv abzuführen.

Wurde im Schritt S1 ein Einschalten des Atemluftbefeuchters 1 erkannt, so kann der (eingeschaltete) Atemluftbefeuchter 1 beispielsweise in einem Schritt S3 direkt im Anschluss an den Betrieb im Vorbereitungsmodus automatisch in den Befeuchtungsmodus umgeschaltet und so lange im Befeuchtungsmodus betrieben werden, bis der Atemluftbefeuchter 1 wieder ausgeschaltet wird. Denkbar ist auch, dass der jeweilige Benutzer nach dem Ende des Vorbereitungsmodus durch Erzeugen eines entsprechenden akustischen und/oder optischen und/oder haptischen Bedienungshinweises mittels des Atemluftbefeuchters 1 und/oder des Beatmungsgeräts dazu aufgefordert wird, den Atemluftbefeuchter 1 in den Befeuchtungsmodus zu schalten.

Wurde im Schritt S1 ein Ausschalten des Atemluftbefeuchters 1 erkannt, so kann der (ausgeschaltete) Atemluftbefeuchter 1 im Anschluss an den Betrieb im Vorbereitungsmodus weiterhin ausgeschaltet bleiben, ohne dass eine weitere Reaktion erfolgt. Es ist also möglich, dass der Atemluftbefeuchter 1- obwohl er ausgeschaltet wurde - (unmittelbar) nach dem Ausschalten noch für eine bestimmte Betriebsdauer im Vorbereitungsmodus betrieben wird, um eine ausreichende Abkühlung des Atemluftbefeuchters 1 zu ermöglichen und/oder die Abkühlung des Atemluftbefeuchters 1 zu beschleunigen, bevor der Atemluftbefeuchter 1 wieder (manuell) eingeschaltet wird, beispielsweise weil der Patient die Beatmungstherapie fortsetzen möchte.

Zweckmäßigerweise kann der Atemluftbefeuchter 1 eine zusätzliche Batterie zur Stromversorgung des Atemluftbefeuchters 1 im ausgeschalteten Zustand, beispielsweise beim Betrieb im Vorbereitungsmodus, umfassen. Die zusätzliche Batterie kann beispielsweise wiederaufladbar sein. In diesem Fall kann der Atemluftbefeuchter 1 ausgebildet sein, um die zusätzliche Batterie im eingeschalteten Zustand, beispielsweise beim Betrieb im Vorbereitungsmodus und/oder im Befeuchtungsmodus, zu laden. Auf diese Weise kann sichergestellt werden, dass die Batterie bei jedem Ausschalten des Atemluftbefeuchters 1 ausreichend geladen ist.

Es ist möglich, dass der Atemluftbefeuchter 1 bei jedem Ein- oder Ausschalten (zuerst) in den Vorbereitungsmodus geschaltet wird. Dabei kann der Atemluftbefeuchter 1 beispielsweise für eine fest vorgegebene Betriebsdauer, vorzugsweise zwischen 30 Sekunden und 5 Minuten, im Vorbereitungsmodus betrieben werden.

Alternativ kann das Schalten in den Vorbereitungsmodus im Schritt S2 temperaturabhängig erfolgen. Zudem ist es möglich, dass die Dauer des Betriebs des Atemluftbefeuchters 1 im Vorbereitungsmodus temperaturabhängig gesteuert wird.

Hierzu kann in einem optionalen Schritt S4 als Reaktion auf das Erkennen des Ein- oder Ausschaltens im Schritt S1 ein Temperaturwert, der eine aktuelle Temperatur der Atemluft im Inneren des Atemluftbefeuchters 1, beispielsweise im Atemluftkanal 7, anzeigt, in der Steuereinheit 15 empfangen werden. Der Temperaturwert kann gemessen und/oder geschätzt sein.

In einem Schritt S5 kann der Temperaturwert anschließend mit einem bestimmten Schwellenwert verglichen werden. Der Schwellenwert zeigt eine Temperaturschwelle an, bei der die Atemluft beim Einatmen gerade noch als angenehm empfunden wird. Der Schwellenwert kann beispielsweise im Speicher 19 gespeichert sein. Der Schwellenwert kann fest vorgegeben sein oder vom jeweiligen Benutzer über eine geeignete Benutzerschnittstelle einstellbar sein.

Ergibt der Vergleich im Schritt S5, dass der Temperaturwert den Schwellenwert überschreitet, d. h., dass die aktuelle Temperatur über der Temperaturschwelle liegt, so kann der Atemluftbefeuchter 1 im Schritt S2 in den Vorbereitungsmodus geschaltet und im Vorbereitungsmodus betrieben werden.

Ergibt der Vergleich im Schritt S5 hingegen, dass der Temperaturwert den Schwellenwert nicht überschreitet, d. h., dass die aktuelle Temperatur mit der Temperaturschwelle übereinstimmt oder unter der Temperaturschwelle liegt, so ist es möglich, dass das Schalten in den Vorbereitungsmodus verhindert wird. Stattdessen kann der Atemluftbefeuchter 1 beispielsweise im Schritt S3 (unmittelbar) nach dem Einschalten in den Befeuchtungsmodus geschaltet und im Befeuchtungsmodus betrieben werden, vorzugsweise so lange, bis der Atemluftbefeuchter 1 wieder ausgeschaltet wird.

Es ist möglich, dass der Temperaturwert im Schritt S4 in mehreren aufeinanderfolgenden Zeitschritten ab dem Erkennen des Ein- oder Ausschaltens im Schritt S1 empfangen wird. Dabei kann der Temperaturwert eines jeden Zeitschritts im Schritt S5 mit dem Schwellenwert verglichen werden. Dies ermöglicht eine temperaturabhängige Steuerung der Dauer des Betriebs im Vorbereitungsmodus, indem der Betrieb im Vorbereitungsmodus automatisch beendet wird, sobald der Temperaturwert den Schwellenwert nicht mehr überschreitet. Beispielsweise kann der (eingeschaltete) Atemluftbefeuchter 1 automatisch vom Vorbereitungsmodus in den Befeuchtungsmodus geschaltet werden, sobald der Temperaturwert den Schwellenwert nicht mehr überschreitet.

Bei dem Temperaturwert kann es sich vorzugsweise um einen bloßen Schätzwert handeln. Dies hat den Vorteil, dass ein gesonderter Temperatursensor zum Erfassen der aktuellen Temperatur im Inneren des Atemluftbefeuchters 1, beispielweise im Atemluftkanal 7, entfallen kann, was die Herstellungskosten reduzieren kann.

Beispielsweise kann der Temperaturwert in einem zusätzlichen Schritt S6 unter Verwendung eines geeigneten mathematischen Modells, das bestimmte physikalische Eigenschaften des Atemluftbefeuchters näherungsweise abbildet, berechnet werden. Hierzu kann mindestens ein gemessener und/oder geschätzter Eingabewert in der Steuereinheit 15 empfangen und in das mathematische Modell eingegeben werden. Das mathematische Modell kann konfiguriert sein, um den mindestens einen Eingabewert in einen entsprechenden Temperaturwert umzuwandeln und den Temperaturwert als einen Ausgabewert auszugeben. Der Ausgabewert kann dann in geeigneter Weise in der Steuereinheit 15 weiterverarbeitet werden.

Der Eingabewert kann beispielsweise eine der folgenden Größen definieren:
- eine aktuelle Heizleistung des Heizelements 9;
- einen aktuellen Füllstand des Behälters 8;
- eine (beispielsweise durchschnittliche) Dauer mindestens einer dem Aus- oder Einschalten vorangegangenen Aufheizphase, in der das Heizelement 9 aktiviert war;
- eine (beispielsweise durchschnittliche) Dauer mindestens einer dem Aus- oder Einschalten vorangegangenen Abkühlphase, in der das Heizelement 9 deaktiviert war;
- eine aktuelle Umgebungstemperatur in der Umgebung des Atemluftbefeuchters 1;
- eine aktuelle relative Luftfeuchtigkeit bezogen auf eine aktuelle Umgebungstemperatur in der Umgebung des Atemluftbefeuchters 1;
- einen aktuellen Umgebungsdruck in der Umgebung des Atemluftbefeuchters 1.

Es ist möglich, dass gleichzeitig mehrere Eingabewerte für mindestens zwei verschiedene Größen, beispielsweise für mindestens zwei der vorstehend aufgelisteten Größen, in das mathematische Modell eingegeben werden, um den Temperaturwert zu berechnen.

Das mathematische Modell kann beispielsweise in Form einer mathematischen Funktion und/oder einer Lookup-Tabelle im Speicher 19 gespeichert sein. Die Parameter des mathematischen Modells können beispielsweise in Versuchen ermittelt worden sein.

Der Betrieb des Atemluftbefeuchters 1 im Vorbereitungsmodus kann sich auch lediglich darauf beschränken, zu verhindern, dass der Atemluftbefeuchter 1 in den Befeuchtungsmodus geschaltet wird, und/oder zu verhindern, dass das Heizelement 9 aktiviert wird. Dies ermöglicht eine kontrollierte passive Wärmeabfuhr.

Zusätzlich oder alternativ ist eine kontrollierte aktive Wärmeabfuhr möglich. Dazu kann die Steuereinheit 15 beispielsweise ein oder mehrere Steuersignale 21 zum Steuern des Aktors 11 (oder der Aktoren 11) während des Betriebs des Atemluftbefeuchters 1 im Vorbereitungsmodus erzeugen.

Mittels des Steuersignals 21 (oder der Steuersignale 21) kann der Aktor 11 (oder können die Aktoren 11) beispielsweise so gesteuert werden, dass:
- ein Atemluftstrom 13 zum Abführen der überschüssigen Wärme durch den Atemlufteingang 3 und/oder durch den Atemluftausgang 5 und/oder durch mindestens eine vom Atemlufteingang 3 und vom Atemluftausgang 5 getrennte Belüftungsöffnung des Atemluftbefeuchters 1 erzeugt wird;
- verhindert wird, dass die Atemluft am Atemluftausgang 5 bereitgestellt wird;
- die Atemluft am Atemluftausgang 5 mit einem geringeren Druck und/oder einem geringeren Volumenstrom und/oder einer anderen Strömungsrichtung als beim Betreiben des Atemluftbefeuchters 1 im Befeuchtungsmodus bereitgestellt wird;
- ein Volumenstrom der am Atemluftausgang 5 bereitgestellten Atemluft einem zumindest teilweise ansteigenden Volumenstromverlauf folgt;
- ein Druck der am Atemluftausgang 5 bereitgestellten Atemluft einem zumindest teilweise ansteigenden Druckverlauf folgt;
- ein Volumenstrom der am Atemluftausgang 5 bereitgestellten Atemluft am Ende des Betriebs des Atemluftbefeuchters 1 im Vorbereitungsmodus einen Endwert erreicht, der gleich einem oder nur geringfügig (beispielsweise höchstens 10 % oder höchstens 5 %) kleiner als ein Volumenstromsollwert ist, den der Volumenstrom zu Beginn eines anschließenden Betriebs des Atemluftbefeuchters 1 im Befeuchtungsmodus haben soll;
- ein Druck der am Atemluftausgang 5 bereitgestellten Atemluft am Ende des Betriebs des Atemluftbefeuchters 1 im Vorbereitungsmodus einen Endwert erreicht, der gleich einem oder nur geringfügig (beispielsweise höchstens 10 % oder höchstens 5 %) kleiner als ein Drucksollwert ist, den der Druck zu Beginn eines anschließenden Betriebs des Atemluftbefeuchters 1 im Befeuchtungsmodus haben soll.

Die in der vorstehenden Liste aufgelisteten Steuerungsmöglichkeiten können einzeln oder in Kombination umgesetzt werden, wobei mindestens zwei der aufgelisteten Steuerungsmöglichkeiten miteinander kombiniert werden können.

Zusätzlich kann im Schritt S2 ein geeigneter Warnhinweis zum Warnen des jeweiligen Benutzers erzeugt werden, beispielsweise davor, dass die Atemluft beim Einatmen möglicherweise als zu feucht und/oder zu warm empfunden wird. Der Warnhinweis kann den jeweiligen Benutzer auch zu einer bestimmten Handlung auffordern, beispielsweise dazu, kurz zu warten und/oder die Beatmungsmaske oder Nasenkanüle abzunehmen, bis die Atemluft ausreichend abgekühlt ist.

Der an den Atemluftausgang 5 angeschlossene Beatmungsschlauch kann (oder die an den Atemluftausgang 5 angeschlossenen Beatmungsschläuche können) optional mittels einer elektrischen Schlauchheizung gezielt beheizbar sein. In diesem Fall kann der Atemluftbefeuchter 1, beispielsweise die Steuereinheit 15, ausgebildet sein, um im Befeuchtungsmodus die durch den Beatmungsschlauch (oder die Beatmungsschläuche) strömende Atemluft durch Steuern der Schlauchheizung zusätzlich zu temperieren. Dementsprechend kann das Verfahren M einen zusätzlichen Schritt umfassen, in dem eine Aktivierung der Schlauchheizung verhindert wird, solange der Atemluftbefeuchter 1 im Vorbereitungsmodus betrieben wird. Auf diese Weise kann eine unerwünschte zusätzliche Erwärmung der im Vorbereitungsmodus durch den Beatmungsschlauch (oder die Beatmungsschläuche) strömenden und möglicherweise bereits zu feuchten und/oder zu warmen Atemluft vermieden werden.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Atemluftbefeuchter
- 3: Atemlufteingang
- 5: Atemluftausgang
- 7: Atemluftkanal
- 8: Behälter
- 9: elektrisches Heizelement
- 11: elektropneumatischer Aktor
- 13: Atemluftstrom
- 15: Steuereinheit
- 17: Prozessor
- 19: Speicher
- 21: Steuersignal
- M: Verfahren
- S1: Erkennen des Ein- oder Ausschaltens
- S2: Schalten in den Vorbereitungsmodus
- S3: Schalten in den Befeuchtungsmodus
- S4: Empfangen eines Temperaturwerts
- S5: Vergleichen des Temperaturwerts
- S6: Berechnen des Temperaturwerts

## Patentansprüche

1. Verfahren (M) zum Betreiben eines Atemluftbefeuchters (1), wobei der Atemluftbefeuchter (1) einen Atemlufteingang (3), einen Atemluftausgang (5) und ein elektrisches Heizelement (9) umfasst, wobei der Atemluftbefeuchter (1) zwischen einem Befeuchtungsmodus und einem Vorbereitungsmodus zum Vorbereiten des Atemluftbefeuchters (1) auf einen Betrieb im Befeuchtungsmodus umschaltbar ist und ausgebildet ist, um im Befeuchtungsmodus Atemluft, die am Atemlufteingang (3) zugeführt wird, zu befeuchten, wobei die Atemluft mittels des Heizelements (9) temperiert wird, und im derart befeuchteten Zustand am Atemluftausgang (5) bereitzustellen, wobei das Verfahren (M) umfasst:
wenn der Atemluftbefeuchter (1) ein- oder ausgeschaltet wird: Schalten (S2) des Atemluftbefeuchters (1) in den Vorbereitungsmodus und Betreiben des Atemluftbefeuchters (1) im Vorbereitungsmodus, um eine Abfuhr überschüssiger Wärme aufgrund eines dem Ein- oder Ausschalten vorangegangenen Betriebs des Atemluftbefeuchters (1) im Befeuchtungsmodus aus dem Inneren des Atemluftbefeuchters (1) zu ermöglichen, wobei das Heizelement (9) deaktiviert ist.

2. Verfahren (M) nach Anspruch 1, ferner umfassend, wenn der Atemluftbefeuchter (1) eingeschaltet wird:
Schalten (S3) des Atemluftbefeuchters (1) in den Befeuchtungsmodus im Anschluss an den Betrieb des Atemluftbefeuchters (1) im Vorbereitungsmodus und Betreiben des Atemluftbefeuchters (1) im Befeuchtungsmodus.

3. Verfahren (M) nach einem der vorhergehenden Ansprüche, ferner umfassend, wenn der Atemluftbefeuchter (1) ein- oder ausgeschaltet wird:
Empfangen (S4) eines Temperaturwerts, der eine aktuelle Temperatur der Atemluft im Inneren des Atemluftbefeuchters (1) anzeigt;
Vergleichen (S5) des Temperaturwerts mit einem Schwellenwert, der eine Temperaturschwelle anzeigt, bei der die Atemluft beim Einatmen gerade noch als angenehm empfunden wird;
wenn der Temperaturwert den Schwellenwert überschreitet: Schalten (S2) des Atemluftbefeuchters (1) in den Vorbereitungsmodus und Betreiben des Atemluftbefeuchters (1) im Vorbereitungsmodus.

4. Verfahren (M) nach Anspruch 3, ferner umfassend, wenn der Atemluftbefeuchter (1) eingeschaltet wird:
wenn der Temperaturwert den Schwellenwert nicht überschreitet: Schalten (S3) des Atemluftbefeuchters (1) in den Befeuchtungsmodus und Betreiben des Atemluftbefeuchters (1) im Befeuchtungsmodus.

5. Verfahren (M) nach Anspruch 3 oder 4,
wobei der Temperaturwert in mehreren aufeinanderfolgenden Zeitschritten empfangen und in jedem Zeitschritt mit dem Schwellenwert verglichen wird, wobei der Atemluftbefeuchter (1) so lange im Vorbereitungsmodus betrieben wird, bis der Temperaturwert den Schwellenwert in einem der Zeitschritte nicht mehr überschreitet.

6. Verfahren (M) nach einem der Ansprüche 3 bis 5, ferner umfassend:
Berechnen (S6) des Temperaturwerts unter Verwendung eines mathematischen Modells des Atemluftbefeuchters (1), wobei mindestens ein Eingabewert empfangen und in das mathematische Modell eingegeben wird, wobei das mathematische Modell den mindestens einen Eingabewert in den Temperaturwert umwandelt und den Temperaturwert als einen Ausgabewert ausgibt, wobei der mindestens eine Eingabewert mindestens eine der folgenden Größen anzeigt:
eine Heizleistung des Heizelements (9);
einen Füllstand eines Behälters (8) zum Speichern einer Flüssigkeit zum Befeuchten der Atemluft;
eine Dauer mindestens einer dem Ein- oder Ausschalten vorangegangenen Aufheizphase, in der das Heizelement (9) aktiviert war;
eine Dauer mindestens einer dem Ein- oder Ausschalten vorangegangenen Abkühlphase, in der das Heizelement (9) deaktiviert war;
eine Umgebungstemperatur in der Umgebung des Atemluftbefeuchters (1);
eine relative Luftfeuchtigkeit bezogen auf eine Umgebungstemperatur in der Umgebung des Atemluftbefeuchters (1);
einen Umgebungsdruck in der Umgebung des Atemluftbefeuchters (1).

7. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), um einen Luftstrom (13) zum Abführen der überschüssigen Wärme durch den Atemlufteingang (3) und/oder durch den Atemluftausgang (5) und/oder durch mindestens eine vom Atemlufteingang (3) und vom Atemluftausgang (5) getrennte Belüftungsöffnung des Atemluftbefeuchters (1) zu erzeugen.

8. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), um zu verhindern, dass die Atemluft am Atemluftausgang (5) bereitgestellt wird; und/oder
Erzeugen eines Steuersignals (21) zum Steuern mindestens eines elektropneumatischen Aktors (11), um die Atemluft am Atemluftausgang (5) mit einem geringeren Druck und/oder einem geringeren Volumenstrom und/oder einer anderen Strömungsrichtung als beim Betreiben des Atemluftbefeuchters (1) im Befeuchtungsmodus bereitzustellen.

9. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), sodass ein Volumenstrom der am Atemluftausgang (5) bereitgestellten Atemluft einem zumindest teilweise ansteigenden Volumenstromverlauf folgt und/oder ein Druck der am Atemluftausgang (5) bereitgestellten Atemluft einem zumindest teilweise ansteigenden Druckverlauf folgt.

10. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), sodass ein Volumenstrom der am Atemluftausgang (5) bereitgestellten Atemluft am Ende des Betriebs des Atemluftbefeuchters (1) im Vorbereitungsmodus einen Endwert erreicht, der gleich einem oder kleiner als ein Volumenstromsollwert ist, den der Volumenstrom zu Beginn eines anschließenden Betriebs des Atemluftbefeuchters (1) im Befeuchtungsmodus haben soll; und/oder
Erzeugen eines Steuersignals (21) zum Steuern mindestens eines elektropneumatischen Aktors (11), sodass ein Druck der am Atemluftausgang (5) bereitgestellten Atemluft am Ende des Betriebs des Atemluftbefeuchters (1) im Vorbereitungsmodus einen Endwert erreicht, der gleich einem oder kleiner als ein Drucksollwert ist, den der Druck zu Beginn eines anschließenden Betriebs des Atemluftbefeuchters (1) im Befeuchtungsmodus haben soll.

11. Verfahren (M) nach einem der vorhergehenden Ansprüche,
wobei der Atemluftbefeuchter (1) ferner einen mittels einer elektrischen Schlauchheizung beheizbaren Beatmungsschlauch zum Anschließen an den Atemluftausgang (5) umfasst;
wobei das Verfahren (M) ferner umfasst: Verhindern einer Aktivierung der Schlauchheizung, solange der Atemluftbefeuchter (1) im Vorbereitungsmodus betrieben wird.

12. Steuereinheit (15), die konfiguriert ist, um das Verfahren (M) nach einem der vorhergehenden Ansprüche auszuführen.

13. Atemluftbefeuchter (1), umfassend einen Atemlufteingang (3), einen Atemluftausgang (5), ein elektrisches Heizelement (9) und eine Steuereinheit (15) nach Anspruch 12, wobei der Atemluftbefeuchter (1) zwischen einem Befeuchtungsmodus und einem Vorbereitungsmodus zum Vorbereiten des Atemluftbefeuchters (1) auf einen Betrieb im Befeuchtungsmodus umschaltbar ist und ausgebildet ist, um im Befeuchtungsmodus Atemluft, die am Atemlufteingang (3) zugeführt wird, zu befeuchten, wobei die Atemluft mittels des Heizelements (9) temperiert wird, und im derart befeuchteten Zustand am Atemluftausgang (5) bereitzustellen.

14. Computerprogramm, umfassend Befehle, die die Steuereinheit (15) nach Anspruch 12 beim Ausführen des Computerprogramms durch die Steuereinheit (15) veranlassen, das Verfahren (M) nach einem der Ansprüche 1 bis 11 auszuführen.

15. Computerlesbares Medium, auf dem das Computerprogramm nach Anspruch 14 gespeichert ist.

## Geänderte Patentansprüche

### Geänderte Patentansprüche gemäss Regel 137(2) EPÜ.

1. Verfahren (M) zum Betreiben eines Atemluftbefeuchters (1), wobei der Atemluftbefeuchter (1) einen Atemlufteingang (3), einen Atemluftausgang (5) und ein elektrisches Heizelement (9) umfasst, wobei der Atemluftbefeuchter (1) zwischen einem Befeuchtungsmodus und einem Vorbereitungsmodus zum Vorbereiten des Atemluftbefeuchters (1) auf einen Betrieb im Befeuchtungsmodus umschaltbar ist und ausgebildet ist, um im Befeuchtungsmodus Atemluft, die am Atemlufteingang (3) zugeführt wird, zu befeuchten, wobei die Atemluft mittels des Heizelements (9) temperiert wird, und im derart befeuchteten Zustand am Atemluftausgang (5) bereitzustellen, wobei das Verfahren (M) umfasst:
Berechnen (S6) eines Temperaturwerts, der eine aktuelle Temperatur der Atemluft im Inneren des Atemluftbefeuchters (1) anzeigt, unter Verwendung eines mathematischen Modells des Atemluftbefeuchters (1), wobei mindestens ein Eingabewert empfangen und in das mathematische Modell eingegeben wird, wobei das mathematische Modell den mindestens einen Eingabewert in den Temperaturwert umwandelt und den Temperaturwert als einen Ausgabewert ausgibt;
wobei das Verfahren (M) ferner umfasst, wenn der Atemluftbefeuchter (1) ein- oder ausgeschaltet wird:
Empfangen (S4) des Temperaturwerts;
Vergleichen (S5) des Temperaturwerts mit einem Schwellenwert, der eine Temperaturschwelle anzeigt, bei der die Atemluft beim Einatmen gerade noch als angenehm empfunden wird;
wenn der Temperaturwert den Schwellenwert überschreitet: Schalten (S2) des Atemluftbefeuchters (1) in den Vorbereitungsmodus und Betreiben des Atemluftbefeuchters (1) im Vorbereitungsmodus, um eine Abfuhr überschüssiger Wärme aufgrund eines dem Ein- oder Ausschalten vorangegangenen Betriebs des Atemluftbefeuchters (1) im Befeuchtungsmodus aus dem Inneren des Atemluftbefeuchters (1) zu ermöglichen, wobei das Heizelement (9) deaktiviert ist;
wobei das Verfahren (M) ferner umfasst, wenn der Atemluftbefeuchter (1) eingeschaltet wird und der Temperaturwert den Schwellenwert nicht überschreitet:
Schalten (S3) des Atemluftbefeuchters (1) in den Befeuchtungsmodus und Betreiben des Atemluftbefeuchters (1) im Befeuchtungsmodus.

2. Verfahren (M) nach Anspruch 1,
wobei der Temperaturwert in mehreren aufeinanderfolgenden Zeitschritten empfangen und in jedem Zeitschritt mit dem Schwellenwert verglichen wird, wobei der Atemluftbefeuchter (1) so lange im Vorbereitungsmodus betrieben wird, bis der Temperaturwert den Schwellenwert in einem der Zeitschritte nicht mehr überschreitet.

3. Verfahren (M) nach einem der vorhergehenden Ansprüche,
wobei der mindestens eine Eingabewert mindestens eine der folgenden Größen anzeigt:
eine Heizleistung des Heizelements (9);
einen Füllstand eines Behälters (8) zum Speichern einer Flüssigkeit zum Befeuchten der Atemluft;
eine Dauer mindestens einer dem Ein- oder Ausschalten vorangegangenen Aufheizphase, in der das Heizelement (9) aktiviert war;
eine Dauer mindestens einer dem Ein- oder Ausschalten vorangegangenen Abkühlphase, in der das Heizelement (9) deaktiviert war;
eine Umgebungstemperatur in der Umgebung des Atemluftbefeuchters (1);
eine relative Luftfeuchtigkeit bezogen auf eine Umgebungstemperatur in der Umgebung des Atemluftbefeuchters (1);
einen Umgebungsdruck in der Umgebung des Atemluftbefeuchters (1).

4. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), um einen Luftstrom (13) zum Abführen der überschüssigen Wärme durch den Atemlufteingang (3) und/oder durch den Atemluftausgang (5) und/oder durch mindestens eine vom Atemlufteingang (3) und vom Atemluftausgang (5) getrennte Belüftungsöffnung des Atemluftbefeuchters (1) zu erzeugen.

5. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), um zu verhindern, dass die Atemluft am Atemluftausgang (5) bereitgestellt wird; und/oder
Erzeugen eines Steuersignals (21) zum Steuern mindestens eines elektropneumatischen Aktors (11), um die Atemluft am Atemluftausgang (5) mit einem geringeren Druck und/oder einem geringeren Volumenstrom und/oder einer anderen Strömungsrichtung als beim Betreiben des Atemluftbefeuchters (1) im Befeuchtungsmodus bereitzustellen.

6. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), sodass ein Volumenstrom der am Atemluftausgang (5) bereitgestellten Atemluft einem zumindest teilweise ansteigenden Volumenstromverlauf folgt und/oder ein Druck der am Atemluftausgang (5) bereitgestellten Atemluft einem zumindest teilweise ansteigenden Druckverlauf folgt.

7. Verfahren (M) nach einem der vorhergehenden Ansprüche,
wobei das Betreiben des Atemluftbefeuchters (1) im Vorbereitungsmodus umfasst:
Erzeugen eines Steuersignals (21) zum Steuern mindestens eines elektropneumatischen Aktors (11), sodass ein Volumenstrom der am Atemluftausgang (5) bereitgestellten Atemluft am Ende des Betriebs des Atemluftbefeuchters (1) im Vorbereitungsmodus einen Endwert erreicht, der gleich einem oder kleiner als ein Volumenstromsollwert ist, den der Volumenstrom zu Beginn eines anschließenden Betriebs des Atemluftbefeuchters (1) im Befeuchtungsmodus haben soll; und/oder
Erzeugen eines Steuersignals (21) zum Steuern mindestens eines elektropneumatischen Aktors (11), sodass ein Druck der am Atemluftausgang (5) bereitgestellten Atemluft am Ende des Betriebs des Atemluftbefeuchters (1) im Vorbereitungsmodus einen Endwert erreicht, der gleich einem oder kleiner als ein Drucksollwert ist, den der Druck zu Beginn eines anschließenden Betriebs des Atemluftbefeuchters (1) im Befeuchtungsmodus haben soll.

8. Verfahren (M) nach einem der vorhergehenden Ansprüche,
wobei der Atemluftbefeuchter (1) ferner einen mittels einer elektrischen Schlauchheizung beheizbaren Beatmungsschlauch zum Anschließen an den Atemluftausgang (5) umfasst;
wobei das Verfahren (M) ferner umfasst: Verhindern einer Aktivierung der Schlauchheizung, solange der Atemluftbefeuchter (1) im Vorbereitungsmodus betrieben wird.

9. Steuereinheit (15) zum Betreiben eines Atemluftbefeuchters (1), wobei der Atemluftbefeuchter (1) einen Atemlufteingang (3), einen Atemluftausgang (5) und ein elektrisches Heizelement (9) umfasst, wobei der Atemluftbefeuchter (1) zwischen einem Befeuchtungsmodus und einem Vorbereitungsmodus zum Vorbereiten des Atemluftbefeuchters (1) auf einen Betrieb im Befeuchtungsmodus umschaltbar ist und ausgebildet ist, um im Befeuchtungsmodus Atemluft, die am Atemlufteingang (3) zugeführt wird, zu befeuchten, wobei die Atemluft mittels des Heizelements (9) temperiert wird, und im derart befeuchteten Zustand am Atemluftausgang (5) bereitzustellen, wobei die Steuereinheit (15) konfiguriert ist, um das Verfahren (M) nach einem der vorhergehenden Ansprüche auszuführen.

10. Atemluftbefeuchter (1), umfassend einen Atemlufteingang (3), einen Atemluftausgang (5), ein elektrisches Heizelement (9) und eine Steuereinheit (15) nach Anspruch 9, wobei der Atemluftbefeuchter (1) zwischen einem Befeuchtungsmodus und einem Vorbereitungsmodus zum Vorbereiten des Atemluftbefeuchters (1) auf einen Betrieb im Befeuchtungsmodus umschaltbar ist und ausgebildet ist, um im Befeuchtungsmodus Atemluft, die am Atemlufteingang (3) zugeführt wird, zu befeuchten, wobei die Atemluft mittels des Heizelements (9) temperiert wird, und im derart befeuchteten Zustand am Atemluftausgang (5) bereitzustellen.

11. Computerprogramm, umfassend Befehle, die die Steuereinheit (15) nach Anspruch 9 beim Ausführen des Computerprogramms durch die Steuereinheit (15) veranlassen, das Verfahren (M) nach einem der Ansprüche 1 bis 8 auszuführen.

12. Computerlesbares Medium, auf dem das Computerprogramm nach Anspruch 11 gespeichert ist.
